# EUROPEAN PATENT SPECIFICATION

(11) **EP 1 412 727 B1**
(45) Date of publication and mention of the grant of the patent: **24.08.2011**
(21) Application number: 00986356.4
(22) Date of filing: 12.12.2000
(51) Int. Cl.: G01N 33/68

(54) **METHOD FOR INTERPRETING TANDEM MASS SPECTROMETRY DATA FOR CLINICAL DIAGNOSIS**
VERFAHREN ZUM INTERPRETIEREN VON TANDEM-MASSENSPEKTROMETERDATEN ZUR KLINISCHEN DIAGNOSE
PROCEDE D'INTERPRETATION DE DONNEES DE SPECTROMETRIE DE MASSE EN TANDEM A DES FINS DE DIAGNOSTIC CLINIQUE

(43) Date of publication of application: 28.04.2004
(73) Proprietor: PerkinElmer Genetics, Inc., Bridgeville, PA 15017 (US)
(72) Inventor: CHACE, Donald, H., Upper St. Clair, PA 15241 (US)
(74) Representative: Williams, Gareth Owen
(86) International application number: PCT/US2000/033790
(87) International publication number: WO 2002/048697

(56) References cited:
- US-A- 5 072 115
- US-A- 5 251 126
- US-A- 5 365 426
- US-A- 5 453 613
- US-A- 5 545 895
- US-A- 5 878 746
- CHACE D H ET AL: "USE OF PHENYLALANINE-TO-TYROSINE RATIO DETERMINED BY TANDEM MASS SPECTROMETRY TO IMPROVE NEWBORN SCREENING FOR PHENYLKETONURIA OF EARLY DISCHARGE SPECIMENS COLLECTED IN THE FIRST 24 HOURS" CLINICAL CHEMISTRY, AMERICAN ASSOCIATION FOR CLINICAL CHEMISTRY. WINSTON, US, vol. 44, no. 12, 1998, pages 2405-2409, XP002938787 ISSN: 0009-9147
- RASHED MOHAMED S ET AL: "Screening blood spots for inborn errors of metabolism by electrospray tandem mass spectrometry with a microplate batch process and a computer algorithm for automated flagging of abnormal profiles" CLINICAL CHEMISTRY, vol. 43, no. 7, 1997, pages 1129-1141, XP002283467 ISSN: 0009-9147
- CHACE DONALD: 'Use of phenylalanine to tyrosine ratio determined by tandem mass spectrometry...in the first 24 hours' CLINICAL CHEMISTRY vol. 44, no. 12, 1998, pages 2405 - 2409, XP002938787

## Description

### TECHNICAL FIELD:

Interpreting data used for clinical diagnostic purposes. In particular, a method for interpreting electrospray tandem mass spectrometry data relating to the quantification of metabolites used for diagnosing newborn babies is disclosed.

### BACKGROUND ART:

Automated methods for assessing a patient's condition are known. Computerized systems can be integrated to produce data that can be compared to a known result to allow for proper diagnosing. Such data might be produced by a MRI or CAT scanner, which is used to identify components within the human body.

One particular instrument used for identifying components of interest, whether they are of medicinal or chemical interest, is the mass spectrometer. In reference to U.S. Patent No. 5,453,613, compounds, when introduced to the electrospray tandem mass spectrometer, are ionized and essentially fragmented. Each fragment produces a peak having local maximums that are matched to reference spectra. A compound can be identified by its associated fragments, each having a mass to charge ratio, which is then relative to the concentrations of each fragment. All of the reference spectra and compound names can then be stored in a library for correlation and determination. Thus, mass/charge ratios can be used to identify components from known spectra stored in a database.

More recently, however, the use of spectrometry has been implemented in the field of clinical diagnosis. See Chace, U.S. application serial number 09/277,119.

Inborn errors of metabolism usually result from defective enzymes or cofactors. Resulting genetic disorders can be diagnosed by the metabolic profiling of amino acids and acylcarnitines taken from blood spots subjected to a sampling protocol and thereafter introduced into an electrospray tandem mass spectrometer. An electrostpray tandem mass spectrometer is very sensitive and specific and can detect a broad spectrum of disorders at the genetic level. With proper standards, data produced from the spectrometer includes values for particular metabolites. The metabolites that are of interest in detecting these disorders are, in particular, amino acids and acylcamitines/camitines and the derivatives thereof.

The spectra and resulting concentration values of each metabolite, as derived from mass spectrometry, are then compared to thresholds as a means for evaluating the contents of the blood sample. These thresholds determine the appropriate course of action necessary as a follow-up to the spectral analysis.

As seen in Wright et al., 5,545,895, spectrometry data is applied to a computerized search database for matching each component as a means of identification. In a clinical diagnostic setting, there must be further methods for evaluation beyond just that of the identification itself. The numbers must be quantified. Newborns can be born with metabolic disorders, which, if not treated within days, can result in death. Thus, after obtaining MS/MS (tandem mass spectrometer) data from blood samples from newborns, generally of the age of less than seven days old, there is a need for efficiently interpreting this data in relation to pre-determined metabolite concentration thresholds. This interpretation allows for proper decision-making necessary for the diagnosing and follow-up testing of newborns.

Chace D H et al. (Clinical Chemistry, 44:12, 1998) describe a method for screening and diagnosing phenylketonuria in newborn infants. More specifically, Chase et al., describe using tandem mass spectrometry to quantify phenylalanine and tyrosine levels in filter paper blood spots collected from newborn infants under 24 hours of age.

Rashed M S et al. (Clinical Chemistry, 43:7, 1997) describe the development and evaluation of a microplate-based batch method for preparing blood-spot samples and the development of a computer algorithm for automated flagging of abnormal metabolic profiles by using tandem mass spectrometry-derived diagnostic parameters for amino acids and acylcarnitines in newborns.

### DISCLOSURE OF INVENTION:

The objective of the present invention is to provide a method for interpreting electrospray tandem mass spectrometry data from the steps following analysis to diagnosis. Thus the present invention provides a method for assisting in a diagnosis of propionic acidemia by interpreting data produced after a dry blood spot on filter paper is derivatized and scanned by a tandem mass spectrometer, comprising the steps of:
acquiring said data, wherein said data includes values comprised of a propionyl carnitine concentration (C3), a molar ratio of an mrm scan of propionyl carnitine and acetyl carnitine (C3mrm/C2) and a molar ratio of propionyl carnitine and palmitoyl carnitine (C3/C16);
applying said values to a propionyl carnitine (C3) decision tree, wherein said decision tree comprises a C3 flag threshold, a C3mrm/C2 flag threshold, and a C3/C16 flag threshold as diagnostic limits to said C3, said C3mrm/C2, and said C3/C16 respectively;
comparing said values, respectively, with each of said flag thresholds;
identifying whether or not there is an elevation of any of said values above any of said flag thresholds;
interpreting said sample as being normal for said propionyl carnitine, provided there is no said elevation of any of said values;
performing a subsequent re-analysis to obtain mean values, provided any of the following occurs:
   i. said C3 is equal to or greater than said C3 flag threshold;
   ii. said C3/C16 is greater than or equal to said C3/C16 flag threshold and said C3/C2 is greater than said C3/C2 flag threshold and said C3 is greater than about 2.5 µM; or
   iii. said C3 is greater than about 4 µM, and either said C3/C16 is greater than said
      C3/C16 flag threshold or said C3mrm/C2 is greater than said C3mrm/C2 flag threshold;
optionally, performing an immediate re-analysis with a preliminary follow-up to obtain priority mean values, provided any of the following occurs:
   i. said C3 is greater than about 9.0 µM; or
   ii. said C3 is greater than about 7.0 µM and said C3mrm/C2 is greater than said C3mrm/C2 flag threshold or said C3/C16 is greater than said C3/C16 flag threshold; and
initiating a follow-up protocol based on follow-up criteria utilizing said mean values or said priority mean values for diagnostic purposes provided either said subsequent reanalysis or said immediate re-analysis is performed.

The method provides the next course of action necessary in determining the deficiency or evaluation of a particular fragment directly proportional to a concentration of the metabolite, propionyl carnitine, that may cause a genetic defect. In accordance with U.S. application Ser. No. 09/277,119, when an abnormal sample is flagged after being scanned, a recommended action is to be taken. The present method is a guideline for the necessary action following the preliminary analysis.

Internal standards are used to provide the quantitative information needed to detect specific components. Use of proper ratios of respective ions enables the detection of many metabolites at one time. Each particular metabolite is produced as a fragment yielding a concentration within the spectrometer after being quantified and derivatized from a blood spot. Each metabolite concentration is compared to a flag concentration, which is a quality assurance indicator used to identify a proper sampling quantification and analysis, and which is a diagnostic limit in determining whether or not the concentration of the metabolite is significant The flag is pre-determined based on a standard deviation from what a normal concentration of a particular metabolite should be. This concentration threshold, or flag, must be appropriated for each scan done and for each type of metabolite reviewed. The concentration values produced will be above or below this threshold flag, which allows for the determination of the next course of action, whether it be a re-analysis or the interpretation that the baby is normal.

As an example, medium chain acyl-CoA dehydrogenase (MCAD) deficiency could be a result of an increased concentration of octanoylcarnitine (Chace et al.). Deficiency in the activity of MCAD presents with a Reye-like syndrome, mild hypoglycemia, or sudden death. A method described herein provides for numerical guidelines for determining just how significant the elevation is at the time of birth, and what the next step in the screening process would be, such as a follow-up and confirmatory DNA test. In this manner, decision trees for interpreting the concentrations for amino acids and acylcarnitine/carnitines are described, some of which, if properly diagnosed, can lead to treatment. Each of these potentially fatal blood elevations or deficiencies is compared against the quantified concentration thresholds to allow for immediate attention and action. The comparison with the threshold flags also is a determining factor for maintaining instrument quality and accuracy. This decision making process, coupled with the current method of screening newborns using electrospray tandem MS/MS, allows for a complete protocol for analyzing and diagnosing newborns with genetic disorders.

### BRIEF DESCRIPTION OF THE DRAWINGS:

FIG. 1 is a flow diagram of the overall methodology representing the major steps involved from analysis to diagnosis.
FIG. 2 is a flow diagram showing the more detailed steps and decision tree involved in the re-analysis protocol.
FIG. 3 is a flow diagram showing the more detailed steps and decision tree involved in the follow-up protocol.
FIG. 4 is the decision tree for the implementation of the method for propionyl carnitine.
FIG. 5 is the decision tree for the implementation of the method for isovaleryl carnitine.
FIG. 6 is the decision tree for the implementation of the method for methionine.
FIG. 7 is the decision tree for the implementation of the method for glutaryl carnitine.
FIG. 8 is the decision tree for the implementation of the method for phenylalanine.
FIG. 9 is the decision tree for the implementation of the method for leucine.
FIG. 10 is the decision tree for the implementation of the method for citrulline.
FIG. 11 is the decision tree for the implementation of the method for octanoyl carnitine.
FIG. 12 is the decision tree for the implementation of the method for myristoyl carnitine.
FIG. 13 is the decision tree for the implementation of the method for hydroxy-C5.

### BEST MODE FOR CARRYING OUT THE INVENTION:

The method will now be described in detail in relation to a preferred embodiment and implementation thereof which is exemplary in nature and descriptively specific as disclosed. As is customary, it will be understood that no limitation of the scope of the invention is thereby intended. The invention encompasses such alterations and further modifications in the illustrated device, and such further applications of the principles of the invention illustrated herein, as would normally occur to persons skilled in the art to which the invention relates.

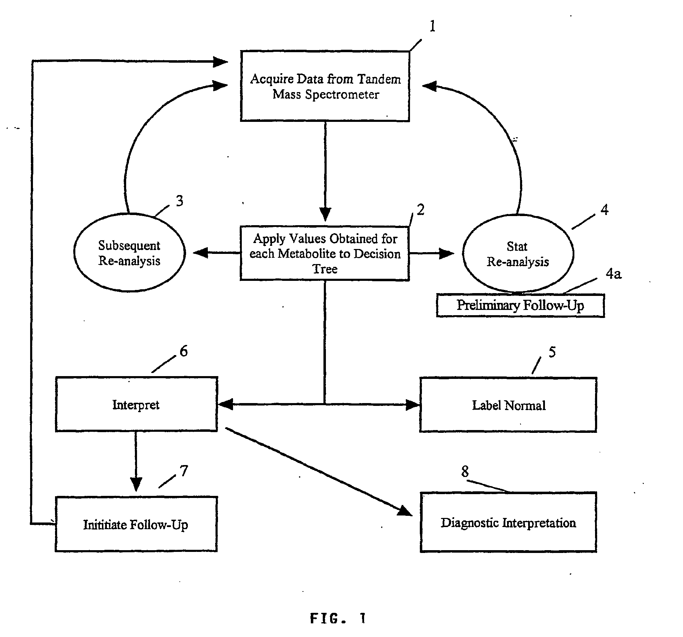

The steps in the overall methodology are shown in FIG. 1. After the blood samples are scanned by the electrospray tandem mass spectrometer, the data is acquired **1.** This data can be presented on a monitor to allow for viewing and/or printing of the output. As part of this data acquiring **1,** the first values produced from the scan of the mass spectrometer are processed and printed into spreadsheet form to further allow checking of the calculations, a means of assuring accurate number production and quality. The acquired data **1** is then examined by applying the first values obtained to the decision tree **2** particular to a metabolite. The first values to be interpreted **2** consist of mean metabolite concentrations and molar ratio concentrations produced from the fragmentation of the metabolites scanned. The concentration of the particular metabolite is read from the fragmentation of the butyl ester occurring at a specific mass to charge value upon derivatization of the metabolite. This quantified number, normally in units of micromolarity, is compared to the flag threshold.

The metabolites, as further described, are grouped as carnitines/acylcarnitines or amino acids. Each particular metabolite is derivatized to enhance the detection of the fragment of concern, and would produce a peak upon scanning corresponding to a quantified concentration number, and compared to the decision tree **2** flag threshold, which is a particular standard deviation away from a mean value for the particular metabolite fragment concentration. The flag threshold particular for that metabolite is a diagnostic limit to these first values.

When the first values for the flags and metabolite fragment concentrations or molar ratio concentrations are applied to the decision tree **2,** it is identified whether or not there needs to be a subsequent re-analysis **3,** or an immediate, stat re-analysis **4.** A subsequent analysis **3** may be necessarily performed if the scan revealed a mean concentration that is equal to or slightly greater than the pre-determined threshold for the flag setting. Each threshold is unique for a particular metabolite concentration, as further described. It should be understood that a concentration is deemed relevant if it exceeds a threshold when the metabolite may cause defects if elevated, and the concentration is relevant if it is below the threshold when the metabolite of question may cause defects if deficient. Because of this fact, any elevation or deficiency can be called a deviation. For the purposes of clarification, an elevation will be discussed, whereby a deficiency should be inherently understood depending on the metabolite. Both upper and lower concentration flag thresholds and molar ratio thresholds may be utilized in some cases where both an elevation and deficiency is significant in determining what defect may be present.

A stat re-analysis **4** may be performed if an initial concentration of the metabolite *significantly* deviates from the flag threshold, which may be evident of a genetic disorder. An immediate preliminary follow-up **4a** would then follow. Whether or not the deviation is deemed significant depends on the amount by which the metabolite concentration deviates from the flag threshold based on the interpretation guide for each disorder. In any effect, a subsequent analysis **3** or a stat re-analysis **4** requires the data to be re-acquired **1**. If the scan shows normal metabolite concentration levels as compared to the concentration flag thresholds and molar ratio thresholds, the interpretation may be that the blood component levels are normal **5**.

Depending on which particular metabolite is being scanned, if the metabolite concentration is not significantly above the threshold, but still deviates in relation to the threshold, even after re-analysis, the next step would be to interpret **6** the sample as being evident of this elevation (or deficiency). In this case, a follow-up protocol **7** would be initiated, such that the detection process would be repeated and all attention would be focused on this sample. After any subsequent repeat, a diagnostic interpretation **8** may follow if the first concentration is consistent with any subsequent evaluation.

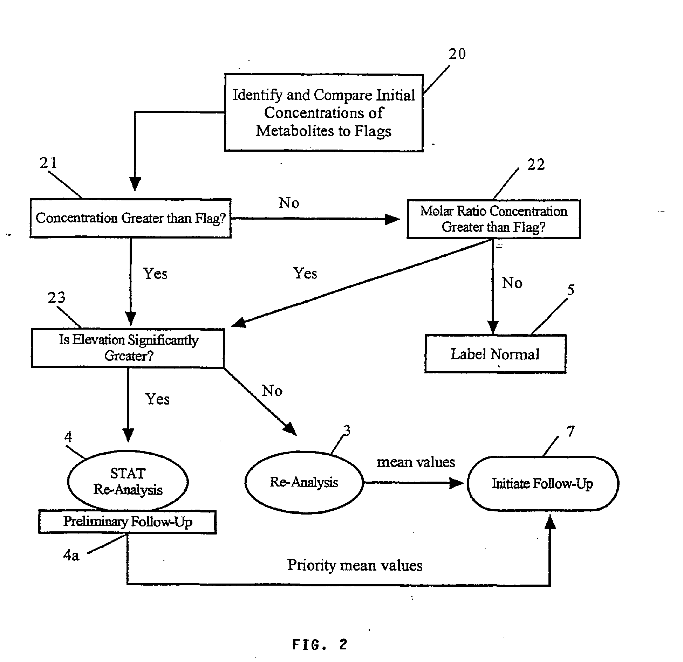

The criteria involved for deciding whether or not to subsequently re-analyze **3,** or to immediately (stat) re-analyze **4** with an immediate preliminary follow-up **4a,** are further described in relation to FIG. 2. The pre-determined threshold flags for each particular metabolite are compared to the initial concentrations of the metabolites **20** after being scanned. The threshold flag settings have been developed based upon many factors such as published reports, the clinical screening of individuals who are already sick, autopsy reports, and experience through repetition of genetic data analysis. If the initial concentration of the metabolite of interest is greater than the flag threshold **21,** then a determination is made as to how significant the elevation is **23.** This is determined by comparing each flag threshold to the initial concentration of the metabolite and identifying whether or not the elevation (or deficiency) exceeds the flag threshold by reference to criteria for each metabolite. If this were to occur, an immediate stat re-analysis **4** and preliminary follow-up **4a** would be performed. This is achieved by prioritizing the sample to allow for an immediate preliminary follow-up and re-running the sample from the same filter card to acquire a set of prioritized values. These values are then averaged as a mean with the first values to form priority mean values because they are more significant for implementation into the follow-up protocol.

If the initial concentration of the metabolite is not greater than the flag threshold **21,** the molar ratio concentrations are identified and compared to their respective concentration flag thresholds and molar ratio thresholds **22.** The molar ratios are important because they account for variability of the blood spot on the filter card. Because the sample is originally dry, the variability of the thickness, number of cells, and change in volume must be accounted for. Thus, as the concentration of the metabolite may go up or down, the ratio of two analytes in one sample is a more sensitive indicator because they both change relative to one another.

If the molar ratio concentrations are not greater than the concentration flag thresholds and molar ratio thresholds **22,** then the sample may be labeled as normal. However, if the molar ratio concentrations are found to be greater **22** by reference to the criteria particular for that metabolite, the elevation significance is then compared as above **23.** Thus, if either the initial concentration of the metabolite or the molar ratios are significantly elevated by reference to the criteria, an immediate stat re-analysis **4** with preliminary follow-up **4a** is performed. And in both cases, if the elevation is present by comparison to the flag threshold **21** but not significant **23,** a sub-sequent re-analysis **3** is performed. This is achieved by acquiring a new set of data to obtain second values from the sample, by re-testing the sample and averaging the results to form mean values of the concentrations, which can then be implemented into the follow-up protocol, as follows.

A follow-up protocol is then initiated **7** following any re-analysis for determining any possible interpretation for diagnosis.

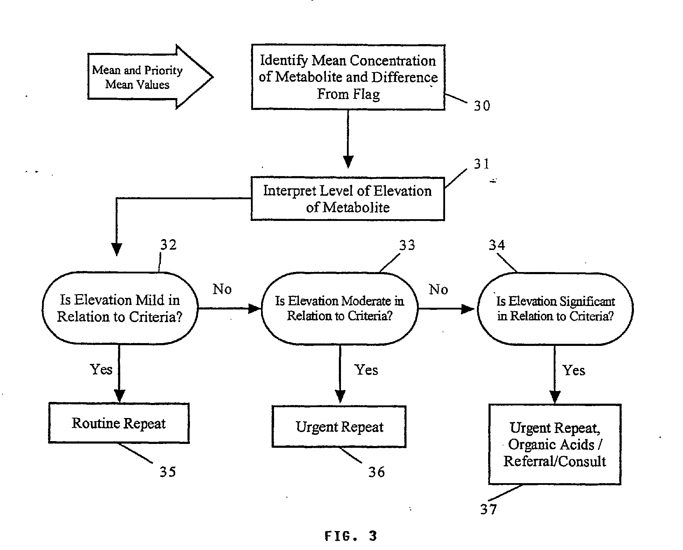

FIG. 3 lays out the criteria for broadly initiating a follow-up **7.** Any mean values or priority mean values are identified from the subsequent re-analysis **3** or the immediate re-analysis **4,** respectively. These values that are elevated to some degree (dependent on decision matrix) above the flag threshold are identified **30.** This follow-up **7** may also follow any re-analysis. The level of elevation (or deficiency, if required) is interpreted **31** as being mild **32,** moderate **33,** or significant **34** based on the criteria in the interpretation guide for each metabolite, as further described. If the elevation is considered mild **32** based on the difference from the threshold, a routine repeat **35** is performed. This involves performing a less prioritized repeat of the testing without an alert to a parent or physician.

If the elevation is considered moderate **33** based on the criteria, an urgent repeat **36** is performed, which is different from a routine repeat because the elevation is reported to a physician. Accordingly, the physician can suggest to see the baby and possibly get another sample or confirm the results.

If the elevation is considered significant **34** based on the criteria, an urgent repeat plus additional testing to obtain a third set of values, such as for propionic acidemia, or other organic acidemias, is performed along with a referral to a specialist particular to a disorder to ascertain an expertise for clinical evaluation.

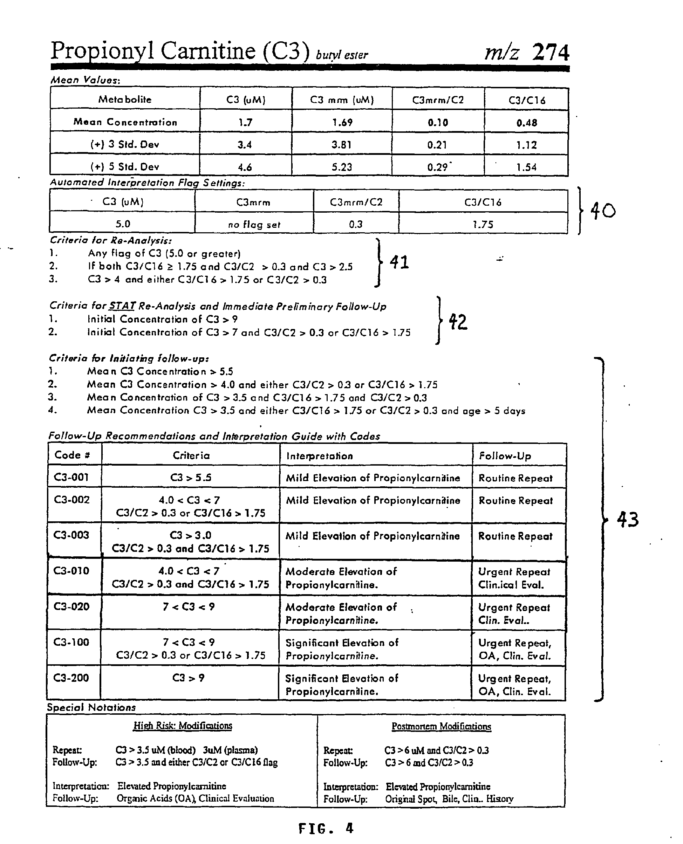

The method described herein can be further understood by referencing the tables and criteria in figures 4-10. Each figure represents the decision tree presented for each particular metabolite, which is necessary because each metabolite ionizes a butyl ester fragment at a different mass to charge (m/z) value, and each metabolite concentration (in units micromolarity [uM]) must be compared to a different threshold flag. The butyl ester fragment is directly proportional to the concentration of the metabolite. The preferred values of the threshold flags, or automated interpretation flag settings, are seen in tabulated form for each metabolite.

FIG. 4 is used in the method for assisting in the diagnosis of propionic acidemia after a dry blood spot on filter paper is derivatized and scanned using a tandem mass spectrometer. The method as previously discussed is applied using the flag thresholds **40** for all concentrations and molar ratios used for determining the level of elevation of propionyl carnitine. The flag thresholds **40** are preferably set at about 5.0 uM for the propionyl carnitine (C3) concentration, about 0.3 for the mrm scan of propionyl carnitine with acetyl carnitine (C3mrm/C2), and about 1.75 for the molar ratio of the propionyl carnitine with palmitoyl carnitine (C3/C16).

The criteria for re-analysis **41** and criteria for an immediate, or STAT re-analysis **42** in relation to the flag thresholds **40** are also shown. A subsequent re-analysis to obtain mean values is performed provided any of the following occurs:
i. C3 is equal to or greater than the C3 flag threshold;
ii. C3/C16 is greater than or equal to the C3/C16 flag threshold and C3/C2 is greater than the C3/C2 flag threshold and C3 is greater than about 2.5 uM;
iii. C3 is greater than about 4 uM, and either said C3/C16 is greater than said C3/C16 flag threshold or said C3/C2 is greater than said C3/C2 flag threshold.

An immediate, or STAT re-analysis with a preliminary follow-up to obtain priority mean values is performed provided any of the following occurs:
i. C3 is greater than about 9.0 uM;
ii. C3 is greater than about 7.0 uM and the C3/C2 is greater than the C3/C2 flag threshold, or the C3/C 16 is greater than the C3/C 16 flag threshold.

The procedure is repeated to get mean values for implementation into a specific follow-up protocol **43,** which ultimately leads to the diagnostic assistance for propionic acidemia.

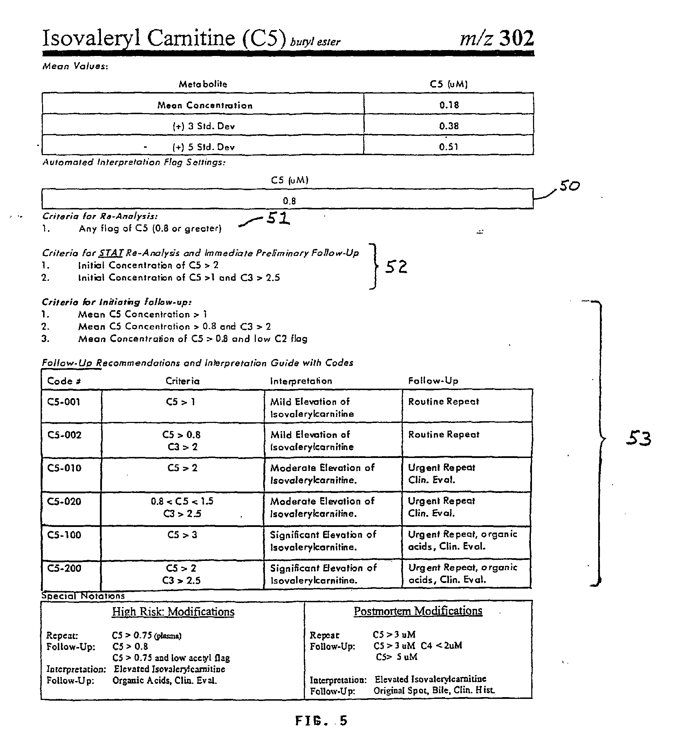

Also described herein, FIG. 5 describes a method for assisting in the diagnosis of isovaleric acidemia after a dry blood spot on filter paper is derivatized and scanned using a tandem mass spectrometer. The method as previously discussed is applied using the flag threshold **50** for the concentration used for determining the level of elevation of isovaleryl carnitine. The flag threshold **50** for isovaleryl carnitine is preferably set at about 0.8 uM.

Criteria for re-analysis **51** and criteria for an immediate, or STAT re-analysis **52** in relation to the flag threshold **50** for isovaleryl carnitine (C5) are also shown. A subsequent re-analysis **51** to obtain a mean value of C5 is performed provided the isovaleryl carnitine concentration (C5) is greater than or equal to the C5 flag threshold **50.**

An immediate re-analysis **52** with a preliminary follow-up to obtain a priority mean value of an isovaleryl carnitine concentration (C5) is performed provided any of the following occurs:
i. the C5 is greater than about 2.0 uM;
ii. the C5 is greater than about 1.0 uM and the propionyl carnitine concentration (C3) [FIG. 4] is greater than about 2.5 uM.

The procedure is repeated to get mean values for implementation into a specific follow-up protocol **53,** which ultimately leads to the diagnostic assistance for isovaleric acidemia.

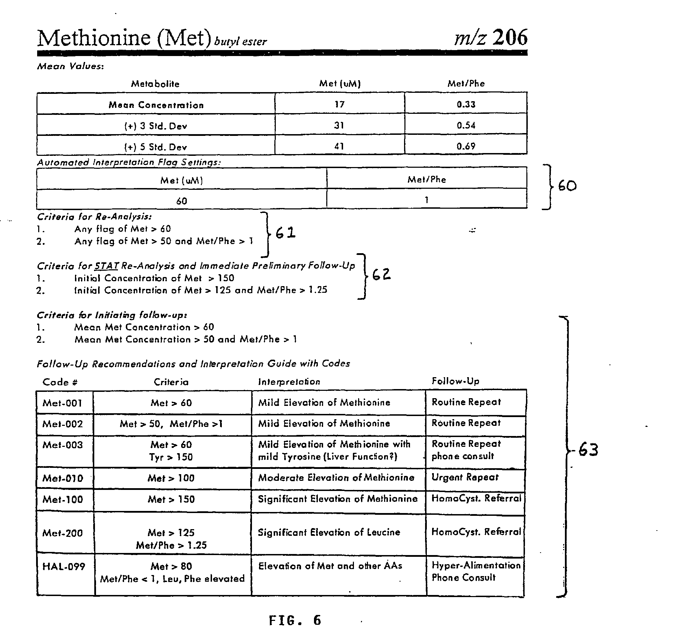

FIG. 6 describes a method for assisting in the diagnosis of hypermethionemia after a dry blood spot on filter paper is derivatized and scanned using a tandem mass spectrometer. The method as previously discussed is applied using the flag thresholds **60** for all concentrations and molar ratios used for determining the level of elevation of methionine. The flag thresholds **60** are preferably set at 60 uM for the methionine concentration (met) and 1 for the molar ratio of methionine and phenylalanine (met/phe).

The criteria for re-analysis **61** and criteria for an immediate, or STAT re-analysis **62** in relation to the flag thresholds are also shown. A subsequent re-analysis to obtain mean values is performed provided any of the following occur:
i. met is greater than the met flag threshold;
ii. met is greater than about 50 uM and the met/phe is greater than the met/phe flag threshold.

An immediate re-analysis with a preliminary follow-up to obtain priority mean values is performed provided any of the following occur:
i. met is greater than about 150 uM;
ii. met is greater than about 125 uM and the met/phe is greater than about 1.25.

The procedure is repeated to get mean values as previously discussed for implementation into a specific follow-up protocol **63,** which ultimately leads to the diagnostic assistance for hypermethionemia.

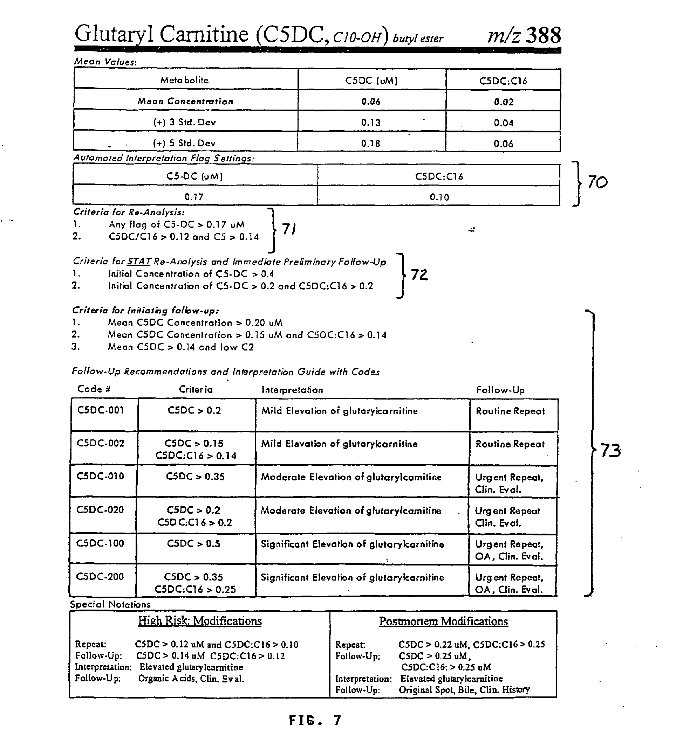

FIG. 7 describes a method for assisting in the diagnosis of glutaric acidemia after a dry blood spot on filter paper is derivatized and scanned using a tandem mass spectrometer. The method as previously discussed is applied using the flag thresholds **70** for all concentrations and molar ratios used for determining the level of elevation of glutaryl carnitine. The flag thresholds **70** are preferably set at 0.17 uM for the glutaryl carnitine concentration (C5DC) and 0.12 for the molar ratio of glutaryl carnitine with palmitoyl carnitine (C5DC:C16).

Criteria for re-analysis **71** and criteria for an immediate, or STAT re-analysis **72** in relation to the flag thresholds are also shown. A subsequent re-analysis **71** to obtain mean values is performed provided any of the following occur:
i. C5DC is greater than the C5DC flag threshold;
ii. C5DC:C16 is greater than the C5DC:C16 flag threshold, and the C5DC is greater than about 0.14 uM.

An immediate re-analysis **72** with a preliminary follow-up to obtain priority mean values is performed provided any of the following occur:
i. C5DC is greater than about 0.4 uM;
ii. C5DC is greater than about 0.2 uM, and the C5DC:C16 is greater than about 0.2 uM.

The procedure is repeated to get mean values as previously discussed for implementation into a specific follow-up protocol **73,** which ultimately leads to the diagnostic assistance for glutaric acidemia.

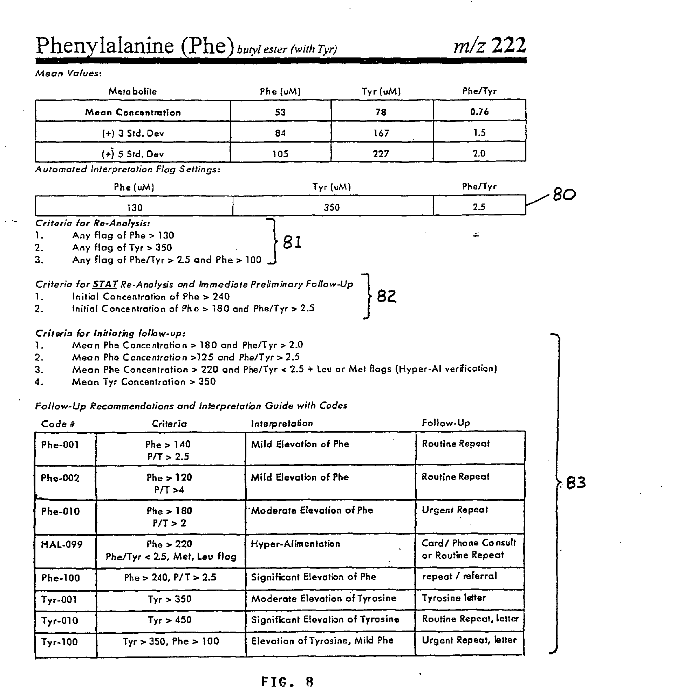

FIG. 8 describes a method for assisting in the diagnosis of phenylketonuria (PKU) after a dry blood spot on filter paper is derivatized and scanned using a tandem mass spectrometer. The method as previously discussed is applied using the flag thresholds **80** for all concentrations and molar ratios used for determining the level of elevation of phenylalanine (phe) or tyrosine (tyr). The flag thresholds **80** are preferably set at 130 uM for the phenylalanine concentration (phe), 350 uM for the tyrosine concentration (tyr), and 2.5 for the molar ratio of phenylalanine with tyrosine (phe/tyr).

The criteria for re-analysis **81** and criteria for an immediate, or STAT re-analysis **82** in relation to the flag thresholds are also shown. A subsequent re-analysis **81** to obtain mean values is performed provided any of the following occur:
i. phe is greater than the phe flag threshold;
ii. tyr is greater than the tyr flag threshold;
iii. phe/tyr is greater than the phe/tyr flag threshold and the phe is greater than about 100 uM.

An immediate re-analysis 82 with a preliminary follow-up to obtain priority mean values is performed provided any of the following occur:
i. phe is greater than about 240 uM;
ii. phe is greater than about 180 uM and the phe/tyr is greater than the phe/tyr flag threshold.

The procedure is repeated to get mean values as previously discussed for implementation into a specific follow-up protocol **83**, which ultimately leads to the diagnostic assistance for PKU.

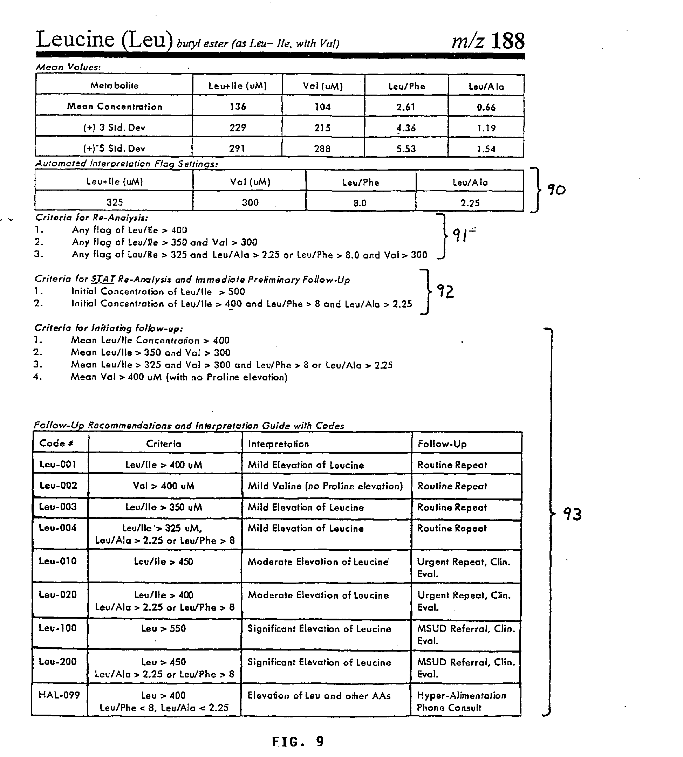

FIG. 9 describes a method for assisting in the diagnosis of Maple Syrup Urine Disease (MSUD) after a dry blood spot on filter paper is derivatized and scanned using a tandem mass spectrometer. The method as previously discussed is applied using the flag thresholds **90** for all concentrations and molar ratios used for determining the level of elevation of leucine. The flag thresholds are preferably set at 325 uM for the concentration of a combination of leucine and isoleucine (leu+Ile); 300 uM for a concentration of valine (val); 8.0 for the molar ratio of leucine with phenylalanine (leu/phe); and 2.25 for the molar ratio of leucine with alanine (leu/ala).

The criteria for re-analysis **91** and criteria for an immediate, or STAT re-analysis **92** in relation to the flag thresholds are also shown. A subsequent re-analysis **91** to obtain mean values is performed provided any of the following occur:
i. leu+ile is greater than about 400 uM;
ii. leu+ile is greater than about 350 uM and val is greater than the val flag threshold;
iii. leu+ile is greater than the leu+ile flag threshold and the leu/ala is greater than the leu/ala flag threshold, or the leu/phe is greater than the leu/phe flag threshold and the val is greater than the val flag threshold;

An immediate re-analysis **92** with a preliminary follow-up to obtain priority mean values is performed provided any of the following occur:
i. leu+ile is greater than about 500 uM;
ii. leu+ile is greater than about 400 uM and the leu/phe is greater than the leu/phe flag threshold and the leu/ala is greater than the leu/ala flag threshold.

The procedure is repeated to get mean values as previously discussed for implementation into a specific follow-up protocol **93,** which ultimately leads to the diagnostic assistance for MSUD.

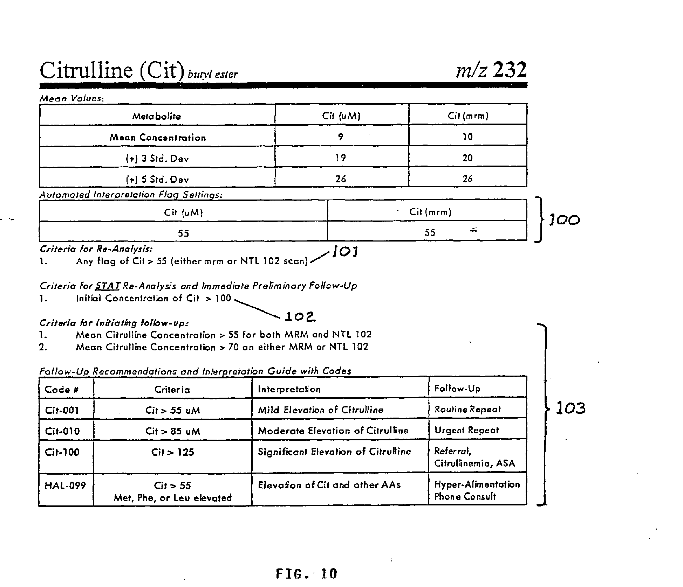

FIG. 10 describes a method for assisting in the diagnosis of citrullinemia after a dry blood spot on filter paper is derivatized and scanned using a tandem mass spectrometer. The method as previously discussed is applied using the flag threshold **100** for the concentrations used for determining the level of elevation of citrulline. The flag threshold **100** is preferably set at 55 uM for a concentration of citrulline determined after a full scan (cit), and 55 uM for a concentration of citrulline determined after an mrm scan (cit[mrm]).

The criteria for re-analysis **101** and criteria for an immediate, or STAT re-analysis **102** in relation to the flag thresholds are also shown. A subsequent re-analysis **101** to obtain mean values is performed provided the following occurs:
i. cit or cit(mrm) is greater than the cit flag threshold or the cit(mrm) flag threshold.

An immediate re-analysis **102** with a preliminary follow-up to obtain priority mean values is performed provided the following occurs:
i. cit is greater than about 100 uM.

The procedure is repeated to get mean values as previously discussed for implementation into a specific follow-up protocol **103,** which ultimately leads to the diagnostic assistance for citrullinemia.

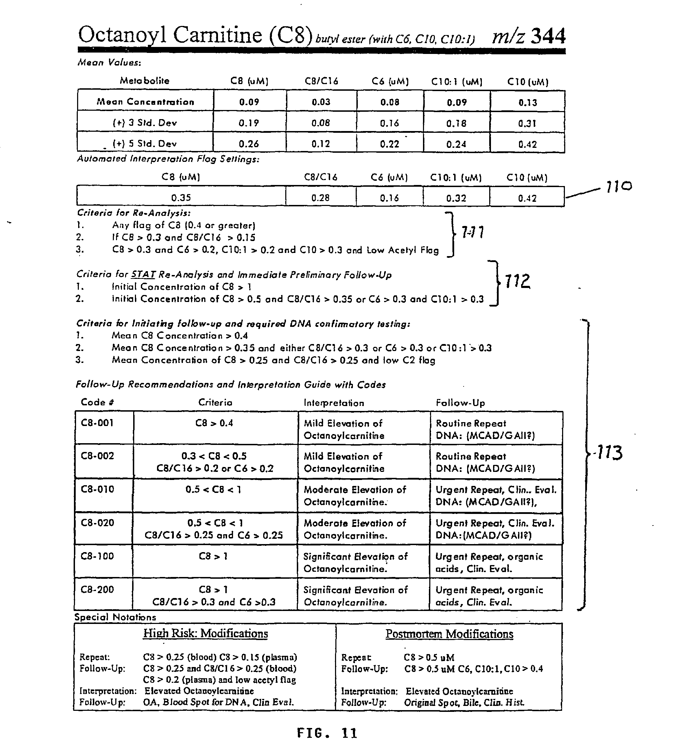

FIG. 11 describes a method for assisting in the diagnosis of medium-chain acyl-coenzyme A dehydrogenase (MCAD) deficiency after a dry blood spot on filter paper is derivatized and scanned using a tandem mass spectrometer. The method as previously discussed is applied using the flag thresholds **110** for all concentrations and molar ratios used for determining the level of elevation of octanoyl carnitine. The flag thresholds **110** are preferably set at 0.35 uM for an octanoyl carnitine concentration (C8); 0.28 for a molar ratio of octanoyl carnitine with palmitoyl carnitine; 0.16 uM for a hexanoyl carnitine concentration (C6); 0.32 uM for a decenoyl carnitine concentration (C10:1); and 0.42 uM for a decanoyl carnitine concentration (C 10).

The criteria for re-analysis **111** and criteria for an immediate, or STAT re-analysis **112** in relation to the flag thresholds **110** are also shown. A subsequent re-analysis **111** to obtain mean values is performed provided any of the following occur:
i. C8 is greater than or equal to about 0.4 uM;
ii. C8 is greater than about 0.3 uM and the C8/C16 is greater than about 0.15;
iii. C8 is greater than about 0.3 uM and the C6 is greater than about 0.2 uM, or the C10:1 is greater than about 0.2 uM and the C10 is greater than about 0.3 uM with a low acetyl flag;

An immediate re-analysis **112** with a preliminary follow-up to obtain priority mean values is performed provided any of the following occur:
i. C8 is greater than about 1.0 uM;
ii. C8 is greater than about 0.5 uM and the C8/C16 is greater than about 0.35, or the C6 is greater than about 0.3 uM and the C 10:1 is greater than about 0.3 uM.

The procedure is repeated to get mean values as previously discussed for implementation into a specific follow-up protocol **113,** which ultimately leads to the diagnostic assistance for MCAD deficiency.

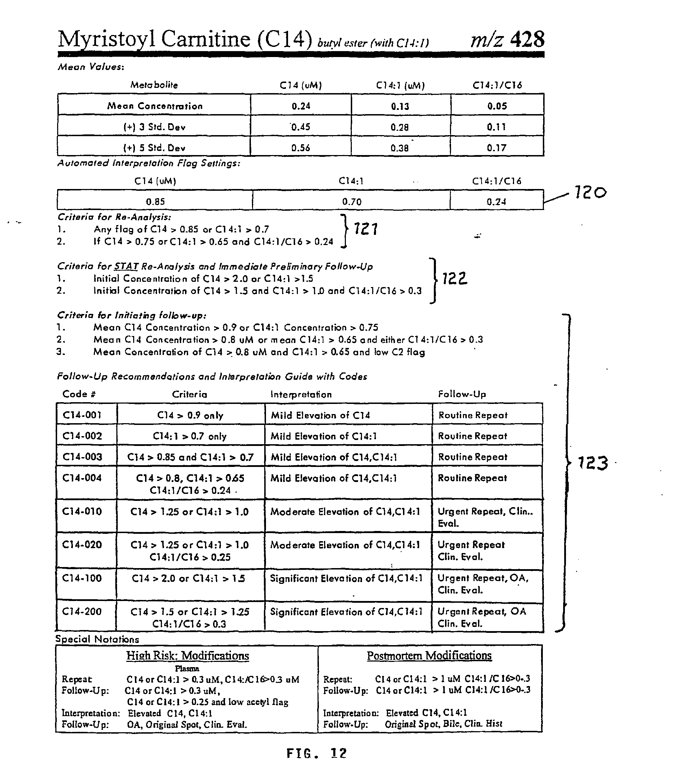

FIG. 12 describes a method for assisting in the diagnosis of very long chain acylCoA dehydrogenase (VLCAD) deficiency after a dry blood spot on filter paper is derivatized and scanned using a tandem mass spectrometer. The method as previously discussed is applied using the flag thresholds **120** for all concentrations and molar ratios used for determining the level of elevation of the myristoylcarnitines. The flag thresholds **120** are preferably set at 0.85 uM for a saturated myristoyl carnitine concentration (C14); 0.70 for an unsaturated myristoyl carnitine concentration (C14:1); and 0.24 for a molar ratio of the unsaturated (C14:1) with palmitoyl carnitine.

The criteria for re-analysis **121** and criteria for an immediate, or STAT re-analysis **122** in relation to the flag thresholds are also shown. A subsequent re-analysis **121** to obtain mean values is performed provided any of the following occur:
i. C 14 is greater than the C 14 flag threshold, or said C14:1 is greater than the C14:1 flag threshold;
ii. C14 is greater than about 0.75 uM, or the C14:1 is greater than about 0.65 uM and the C14:1/C16 is greater than about 0.24.

An immediate re-analysis **122** with a preliminary follow-up to obtain priority mean values is performed provided any of the following occur:
i. C14 is greater than about 2.0 uM, or the C14:1 is greater than about 1.5 uM;
ii. C 14 is greater than about 1.5 uM, and the C14:1 is greater than about 1.0 uM, and the C14:1/C16 is greater than about 0.3.

The procedure is repeated to get mean values as previously discussed for implementation into a specific follow-up protocol **123,** which ultimately leads to the diagnostic assistance for VLCAD deficiency.

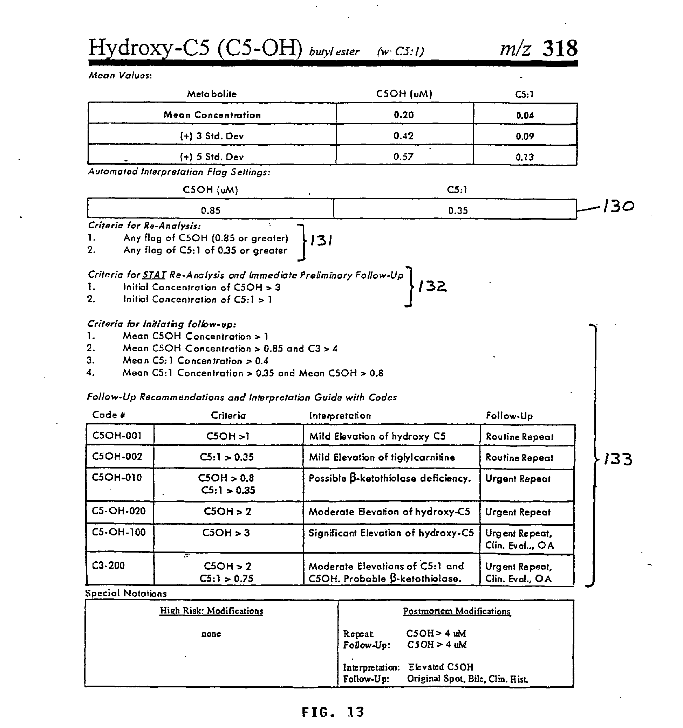

FIG. 13 describes a method for assisting in the diagnosis of crotonyl co-A carboxylase deficiency after a dry blood spot on filter paper is derivatized and scanned using a tandem mass spectrometer. The method as previously discussed is applied using the flag thresholds **130** for all concentrations used for determining the level of elevation of the hydroxy C5. The flag thresholds 130 are preferably set at 0.85 uM for a hydroxy-C5 concentration (C5OH), and 0.35 for a C5:1 concentration.

The criteria for re-analysis **131** and criteria for an immediate, or STAT re-analysis **132** in relation to the flag thresholds are also shown. A subsequent re-analysis **131** to obtain mean values is performed provided any of the following occur:
i. C5OH is greater than or equal to the C5OH flag threshold;
ii. C5:1 is greater than or equal to the C5:1 flag threshold;

An immediate re-analysis **132** with a preliminary follow-up to obtain priority mean values is performed provided any of the following occur:
i. C5OH is greater than about 3.0 uM;
ii. C5:1 is greater than about 1.0 uM.

The procedure is repeated to get mean values as previously discussed for implementation into a specific follow-up protocol **133,** which ultimately leads to the diagnostic assistance for crotonyl coA carboxylase deficiency.

C5OH represents a hydroxy-C5, which is an abbreviated form for hydroxyisovalerylcarnitine and/or hydroyxlmethylbutylcarnitine. The C5:1 is the unsaturated form known as tiglylcarnitine.

In conclusion, according to each decision tree, or interpretation guide particular for each metabolite, the present method allows the data that is acquired to be quantified and reported to a physician to assist in a diagnosis of a genetic disease resulting from the deviation (elevation or deficiency) of a blood metabolite. The method allows one to provide information to a physician that additional tests and/or clinical assessments (check up, examination, etc) are necessary because of the resulting elevation or deficiency of the metabolite, which then results in the confirmed final diagnosis. The values of the flag thresholds for a particular concentration and the molar ratio flag thresholds must be quantified for a consistent and accurate interpretation of acylcarnitine and amino acid data.

### TERMS

It should be understood that "about" as used in relation to Figs 4-13 means ± 15% of the value shown in the criteria for each figure, which values are dependent upon the flag threshold. Any change in the value of the flag threshold, which might result from filter paper characteristics, new insights into disease characterisitics, new diseases found, methods of sample collection, changes in methodology, or corrections from quality assurance programs, would necessitate the relative change of each value in the criteria for all follow-ups. Thus, all values shown in the drawings are the preferred values, which might fluctuate then by no more than ± 15%.

### INDUSTRIAL APPLICABILITY:

The present method quantifies measurements for detecting inborn errors of metabolism usually resulting from defective enzymes or cofactors. Genetic disorders can ultimately be diagnosed by the metabolic profiling of the amino acids and acylcarnitines taken from the blood spots. Newborn babies born with certain metabolic disorders can die if not treated within days. Thus, after obtaining MS/MS (tandem mass spectrometer) data from blood samples from newborns, there is a need for efficiently interpreting this data in relation to a variety of pre-determined metabolite concentration thresholds. This interpretation allows for proper decision-making necessary for the diagnosing and follow-up testing of newborns over a wide range of genetic disorders.

## Claims

1. A method for assisting in a diagnosis of propionic acidemia by interpreting data produced after a dry blood spot on filter paper is derivatized and scanned by a tandem mass spectrometer, comprising the steps of:
acquiring said data, wherein said data includes values comprised of a propionyl carnitine concentration (C3), a molar ratio of an mrm scan of propionyl carnitine and acetyl carnitine (C3mrm/C2) and a molar ratio of propionyl carnitine and palmitoyl carnitine (C3/C16);
applying said values to a propionyl carnitine (C3) decision tree, wherein said decision tree comprises a C3 flag threshold, a C3mrm/C2 flag threshold, and a C3/C16 flag threshold as diagnostic limits to said C3, said C3mrm/C2, and said C3/C16 respectively;
comparing said values, respectively, with each of said flag thresholds;
identifying whether or not there is an elevation of any of said values above any of said flag thresholds;
interpreting said sample as being normal for said propionyl carnitine, provided there is no said elevation of any of said values;
performing a subsequent re-analysis to obtain mean values, provided any of the following occurs:
i. said C3 is equal to or greater than said C3 flag threshold;
ii. said C3/C16 is greater than or equal to said C3/C16 flag threshold and said C3/C2 is greater than said C3/C2 flag threshold and said C3 is greater than about 2.5 µM; or
iii. said C3 is greater than about 4 µM, and either said C3/C16 is greater than said C3/C16 flag threshold or said C3mrm/C2 is greater than said C3mrm/C2 flag threshold;
optionally, performing an immediate re-analysis with a preliminary follow-up to obtain priority mean values, provided any of the following occurs:
i. said C3 is greater than about 9.0 µM; or
ii. said C3 is greater than about 7.0 µM and said C3mrm/C2 is greater than said C3mrm/C2 flag threshold or said C3/C16 is greater than said C3/C16 flag threshold; and
initiating a follow-up protocol based on follow-up criteria utilizing said mean values or said priority mean values for diagnostic purposes provided either said subsequent reanalysis or said immediate re-analysis is performed.

2. The method of claim 1, further comprising:
performing an immediate re-analysis with a preliminary follow-up to obtain priority mean values, provided any of the following occur:
i. said C3 is greater than about 9.0 µM;
ii. said C3 is greater than about 7.0 µM and said C3mrm/C2 is greater than said C3mrm/C2 flag threshold or said C3/C16 is greater than said C3/C16 flag threshold.

3. The method of any one of claims 1 to 2, wherein said C3 flag threshold, said C3/C2 flag threshold, and said C3/C16 flag threshold are, respectively, at least about 3.4 µM, 0.21, and 1.12.

4. The method of any one of claims 1 to 3, wherein said C3 flag threshold, said C3/C2 flag threshold, and said C3/C16 flag threshold are, respectively, at least about 4.6 µM, 0.29, and 1.54.

5. The method of any one of claims 1 to 4, wherein said C3 flag threshold, said C3/C2 flag threshold, and said C3/C16 flag threshold are, respectively, about 5.0 µM, 0.3, and 1.75.

6. A method for assisting in a diagnosis of isovaleric acidemia by interpreting data produced after a dry blood spot on filter paper is derivatized and scanned by a tandem mass spectrometer, comprising the steps of:
acquiring said data, wherein said data includes values comprised of an isovaleryl carnitine concentration (C5) and optionally a propionyl carnitine concentration (C3);
applying said isovaleryl carnitine concentration (C5) to an isovaleryl carnitine (C5) decision tree, wherein said decision tree comprises a C5 flag threshold as diagnostic limit to said isovaleryl carnitine concentration (C5);
comparing said C5 to said C5 flag threshold;
identifying whether or not there is an elevation of C5 above said flag threshold; and
interpreting said sample as being normal for said isovaleryl carnitine, provided there is no said elevation of said C5;
performing a subsequent re-analysis to obtain a mean value of said C5, provided said C5 is greater than or equal to said C5 flag threshold;
optionally, performing an immediate re-analysis with a preliminary follow-up to obtain a priority mean value of said C5, provided any of the following occurs:
i. said C5 is greater than about 2.0 µM; or
ii. said C5 is greater than about 1.0 µM and said C3 is greater than about 2.5 µM; and
initiating a follow-up protocol based on follow up criteria utilizing said mean value or said priority mean value for diagnostic purposes provided either said subsequent re-analysis or said immediate re-analysis is performed.

7. The method of claim 6, further comprising:
performing an immediate re-analysis with a preliminary follow-up to obtain a priority mean value of said C5, provided any of the following occur:
i. said C5 is greater than about 2.0 µM; or
ii. said C5 is greater than about 1.0 µM and said C3 is greater than about 2.5 µM.

8. The method of any one of claims 6 to 7, wherein said C5 flag threshold is at least about 0.38 µM.

9. The method of any one of claims 6 to 8, wherein said C5 flag threshold is at least about 0.51 µM.

10. The method of any one of claims 6 to 9, wherein said C5 flag threshold is at least about 0.8 µM.

## Patentansprüche

1. Verfahren zur Unterstützung einer Diagnose von Propionazidämie durch Interpretation von Daten, die erzeugt werden, nachdem ein trockener Blutfleck auf Filterpapier derivatisiert und durch ein Tandem-Massenspektrometer abgetastet wird, mit den folgenden Schritten:
Erfassen der Daten, wobei die Daten Werte enthalten, die sich aus der Propionylcarnitin-Konzentration (C3), einem Molverhältnis aus einem MRM-Scan von Propionylcarnitin und Acetylcarnitin (C3mrm/C2) und einem Molverhältnis von Propionylcarnitin und Palmitoylcarnitin (C3/C16) zusammensetzen;
Anwendung der Werte auf einen Propionylcarnitin(C3)-Entscheidungsbaum, wobei der Entscheidungsbaum eine C3-Marken-Schwellwert, einen C3mrm/C2-Marken-Schwellwert bzw. einen C3/C16-Schwellwert als diagnostische Grenzwerte für C3, C3mrm/C2 bzw. C3/C16 aufweist;
Vergleichen der jeweiligen Werte mit jedem der Marken-Schwellwerte;
Ermitteln, ob einer der Werte über einen der Marken-Schwellwerte angestiegen ist oder nicht;
Interpretieren der Probe als normal für das Propionylcarnitin unter der Voraussetzung, dass kein Anstieg eines der Werte vorhanden ist;
Durchführen einer späteren Reanalyse, um Mittelwerte zu erhalten, unter der Voraussetzung, dass eines der folgenden Ereignisse eintritt:
i. C3 ist größer oder gleich dem C3-Marken-Schwellwezt;
ii. C3/C16 ist größer oder gleich dem C3/C16-Marken-schwellwert und C3/C2 ist größer als der C3/C2-Maxken-Sckzwellwert und der C3-Wert ist größer als etwa 2,5 µM; oder
iii. C3 ist größer als etwa 4 µM, und entweder C3/C16 ist größer als der C3/C16-Marken-Schwellwert oder C3mrm/C2 ist größer als der C3mrm/C2-Marken-Schwellwert;
wahlweise Durchführen einer sofortigen Reanalyse mit vorläufiger Nachbeobachtung, um Prioritätsmittelwerte zu erhalten, unter der Voraussetzung, dass eines der folgenden Ereignisse eintritt:
i. C3 ist größer als etwa 9,0 µM; oder
ii. C3 ist größer als etwa 7,0 µM and C3mrm/C2 ist größer als der C3mrm/C2-Marken-Schwellwert oder C3/C16 ist größer als der C3/C16-Marken-Schwellwert; und
Initiieren eines Nachbeobachtungsprotokolls, das auf Nachbeobachtungskritezien basiert, welche die Mittelwerte oder die Prioritätsmittelwerte für diagnostische Zwecke nutzen, vorausgesetzt, dass entweder die spätere Reanalyse oder die sofortige Reanalyse durchgeführt wird.

2. Verfahren nach Anspruch 1, das ferner aufweist:
Durchführen einer sofortigen Reanalyse mit vorläufiger Nachbeobachtung, um Prioritätsmittelwerte zu erhalten, unter der Voraussetzung, dass eines der folgenden Ereignisse eintritt:
i. C3 ist größer als etwa 9,0 µM;
ii. C3 ist größer als etwa 7,0 µM, und C3mrm/C2 ist größer als der C3mrm/C2-Marken-Schwettwert, oder C3/C16 ist größer als der C3/C16-Marken-Schwellwert.

3. Verfahren nach einem der Ansprüche 1 bis 2, wobei der C3-Marken-Schwellwert, der C3/C2-Marken-Schwellwert bzw. der C3/C16-Marken-Schwellwert mindestens etwa 3,4, 0,21 bzw. 1,12 µM betragen.

4. Verfahren nach einem der Ansprüche 1 bis 3, wobei der C3-Marken-Schwellwert der C3/C2-Marken-Schwellwert bzw. der C3/C16-Marken-Schwellwert mindestens etwa 4,6, 0,29 bzw. 1,54 µM betragen.

5. Verfahren nach einem der Ansprüche 1 bis 4, wobei der C3-Marken-Schwellwert, der C3/C2-Marken-Schwellwert bzw. der C3/C16-Marken-Schwellwert etwa 5,0, 0,3 bzw. 1,75 µM betragen.

6. Verfahren zur Unterstützung einer Diagnose von Isovalerianazidämie durch Interpretation von Daten, die erzeugt werden, nachdem ein trockener Blutfleck auf Filterpapier derivatisiert und durch ein Tandem-Massenspektrometer abgetastet wird, mit den folgenden Schritten:
Erfassen der Daten, wobei die Daten Werte enthalten, die sich aus der Isovalerylcarnitin-Konzentration (C5) und wahlweise einer Propionylcarnitin-Konzentration (C3) zusammensetzen;
Anwendung der Isovalerylcarnitin-Konzentration (C5) auf einen Isovalerylcarnitin(C5)-Entscheidungsbaum, wobei der Entscheidungsbaum einen C5-Marken-Schwellwert als diagnostischen Grenzwert für die Isovalerylcarnitin-Konzentration (C5) aufweist;
Vergleichen des C5-Werts mit dem C5-Marken-Schwellwert;
Ermitteln, ob C5 über den Marken-Schwellwert angestiegen ist oder nicht; und
Interpretieren der Probe als normal für das Isovalerylcarnitin unter der Voraussetzung, dass kein Anstieg des C5-Werts vorhanden ist;
Durchführen einer späteren Reanalyse, um einen Mittelwert von C5 zu erhalten, unter der Voraussetzung, dass C5 größer oder gleich dem C5-Marken-Schwellwert ist;
wahlweise Durchführen einer sofortigen Reanalyse mit vorläufiger Nachbeobachtung, um einen Prioritätsmittelwert von C5 zu erhalten, unter der Voraussetzung, dass eines der folgenden Ereignisse eintritt:
i. C5 ist größer als etwa 2,0 µM; oder
ii. C5 ist größer als etwa 1,0 µM und C3 ist größer als 2,5 µM; und
Initiieren eines Nachbeobachtungsprotokolls, das auf Nachbeobachtungskriterien basiert, die den Mittelwert oder den Prioritätsmittelwert für diagnostische Zwecke nutzen, vorausgesetzt, dass entweder die spätere Reanalyse oder die sofortige Reanalyse durchgerührt wird.

7. Verfahren nach Anspruch 6, das ferner aufweist:
Durchführen einer sofortigen Reanalyse mit vorläufiger Nachbeobachtung, um einen Prioritätsmittelwert von C5 zu erhalten, unter der Voraussetzung, dass eines der folgenden Ereignisse eintritt:
i. C5 ist größer als etwa 2,0 µM; oder
ii. C5 ist größer als etwa 1,0 µM und C3 ist größer als etwa 2,5 µM.

8. Verfahren nach einem der Ansprüche 6 bis 7, wobei der C5-Marken-Schwellwert mindestens etwa 0,38 µM beträgt.

9. Verfahren nach einem der Ansprüche 6 bis 8, wobei der C5-Marken-Schwellwert mindestens etwa 0,51 µM beträgt.

10. Verfahren nach einem der Ansprüche 6 bis 9, wobei der C5-Marken-Schwellwert mindestens etwa 0,8 µM beträgt.

## Revendications

1. Procédé d'assistance dans un diagnostic d'acidémie propionique par l'interprétation de données produites après qu'un prélèvement de sang sec sur un papier filtre est dérivatisé et balayé par un spectromètre de masse en tandem, comprenant les étapes consistant à :
acquérir lesdites données, lesdites données incluant des valeurs composées d'une concentration en propionyl carnitine (C3), d'un rapport molaire d'un balayage mrm entre propionyl carnitine et acétyl carnitine (C3mrm/C2) et d'un rapport molaire entre propionyl carnitine et palmitoyl carnitine (C3/C16);
appliquer lesdites valeurs à un arbre de décision de propionyl carnitine (C3), ledit arbre de décision comprenant un seuil de drapeau C3, un seuil de drapeau C3mrm/C2, et un seuil de drapeau C3/C16 en tant que limites de diagnostic desdites valeurs C3, C3mrm/C2 et C3/C16, respectivement ;
comparer lesdites valeurs, respectivement, à chacun desdits seuils de drapeau ;
identifier s'il existe ou non une élévation de l'une quelconque desdites valeurs au-dessus de l'un quelconque desdits seuils de drapeau;
interpréter ledit échantillon comme normal pour ladite propionyl carnitine, à condition qu'il n'y ait aucune dite élévation de l'une quelconque desdites valeurs ;
effectuer une nouvelle analyse ultérieure pour obtenir des valeurs moyennes, si l'un quelconque des événements suivants survient :
i. ladite valeur C3 est supérieure ou égale audit seuil de drapeau C3 ;
ii. ledit rapport C3/C16 est supérieur ou égal audit seuil de drapeau C3/C16 et ledit rapport C3/C2 est supérieur audit seuil de drapeau C3/C2 et ladite valeur C3 est supérieure à environ 2,5 µM ; ou
iii. ladite valeur C3 est supérieure à environ 4 µM, et soit ledit rapport C3/C16 est supérieur audit seuil de drapeau C3/C16, soit ledit rapport C3mrm/C2 est supérieur audit seuil de drapeau C3mrm/C2 ;
facultativement, effectuer une nouvelle analyse immédiate avec un suivi préliminaire pour obtenir des valeurs moyennes prioritaires, à condition que l'un quelconque des événements suivants survienne :
i. ladite valeur C3 est supérieure à environ 9,0 µM ; ou
ii. ladite valeur C3 est supérieure à environ 7,0 µM et ledit rapport C3mrm/C2 est supérieur audit seuil de drapeau C3mrm/C3 ou ledit rapport C3/C16 est supérieur audit seuil de drapeau C3/C16 ; et
initier un protocole de suivi d'après des critères de suivi utilisant lesdites valeurs moyennes ou lesdites valeurs moyennes prioritaire à des fins de diagnostic si ladite nouvelle analyse ultérieure ou ladite nouvelle analyse immédiate est effectuée.

2. Procédé selon la revendication 1, comprenant en outre :
la conduite d'une nouvelle analyse immédiate avec un suivi préliminaire pour obtenir des valeurs moyennes prioritaires, à condition que l'un quelconque des événements suivants survienne :
i. ladite valeur C3 est supérieure à environ 9,0 µM ;
ii. ladite valeur C3 est supérieure à environ 7,0 µM et ledit rapport C3mrm/C2 est supérieur audit seuil de drapeau C3mrm/C2 ou ledit rapport C3/C16 est supérieur audit seuil de drapeau C3/C16.

3. Procédé selon l'une quelconque des revendication 1 à 2, dans lequel ledit seuil de drapeau C3, ledit seuil de drapeau C3/C2 et ledit seuil de drapeau C3/C16 sont, respectivement, d'au moins 3,4 µM, 0,21, et 1,12.

4. Procédé selon l'une quelconque des revendications 1 à 3, dans lequel ledit seuil de drapeau C3, ledit seuil de drapeau C3/C2 et ledit seuil de drapeau C3/C16 sont, respectivement, d'au moins environ 4,6 µM, 0,29 et 1,54.

5. Procédé selon l'une quelconque des revendications 1 à 4, dans lequel ledit seuil de drapeau C3, ledit seuil de drapeau C3/C2 et ledit seuil de drapeau C3/C16 sont, respectivement, d'au moins environ 5,0 µM, 0,3 et 1,75.

6. Procédé d'assistance dans un diagnostic d'acidémie isovalérique par l'interprétation de données produites après qu'un prélèvement de sang sec sur un papier filtre est dérivatisé et balayé par un spectromètre de masse en tandem, comprenant les étapes consistant à :
acquérir lesdites données, lesdites données incluant des valeurs composées d'une concentration en isovaléryl carnitine (C5) et facultativement une concentration en propionyl carnitine (C3) ;
appliquer ladite concentration en isovaléryl carnitine (C5) à un arbre de décision de isovaléryl carnitine (C5), ledit arbre de décision comprenant un seuil de drapeau C5 en tant que limite de diagnostic à ladite concentration en isovaléryl carnitine (C5) ;
comparer ladite valeur C5 audit seuil de drapeau C5 ;
identifier s'il existe ou non une élévation de C5 au-dessus dudit seuil de drapeau ; et
interpréter ledit échantillon comme normal pour ladite isovaléryl carnitine à condition qu'il n'y ait aucune dite élévation de ladite valeur C5 ;
effectuer une nouvelle analyse ultérieure pour obtenir une valeur moyenne de ladite valeur C5, à condition que ladite valeur C5 soit supérieure ou égale audit seuil de drapeau C5 ;
facultativement, effectuer une nouvelle analyse immédiate avec un suivi préliminaire pour obtenir une valeur moyenne prioritaire de ladite valeur C5, à condition que l'un quelconque des événements suivants survienne :
i. ladite valeur C5 est supérieure à environ 2,0 µM; ou
ii. ladite valeur C5 est supérieure à environ 1,0 µM et ladite valeur C3 est supérieure à environ 2,5 µM; et
initier un protocole de suivi d'après des critères de suivi utilisant ladite valeur doyenne ou ladite valeur moyenne prioritaire à des fins de diagnostic à condition que soit ladite nouvelle analyse ultérieure soit ladite nouvelle analyse immédiate soit effectuée.

7. Procédé selon la revendication 6, comprenant en outre:
la conduite d'une nouvelle analyse immédiate avec un suivi préliminaire pour obtenir une valeur moyenne prioritaire de ladite valeur C5, à condition que l'un quelconque des événements suivants survienne:
i. ladite valeur C5 est supérieure à environ 2,0 µM; ou
ii. ladite valeur C5 est supérieure à environ 1,0 µM et ladite valeur C3 est supérieure à environ 2,5 µM.

8. Procédé selon l'une quelconque des revendication 6 à 7, dans lequel ledit seuil de drapeau C5 est d'au moins environ 0,38 µM.

9. Procédé selon l'une quelconque des revendications 6 à 8, dans lequel ledit seuil de drapeau C5 est d'au moins environ 0,51 µM.

10. Procédé selon l'une quelconque des revendications 6 à 9, dans lequel ledit seuil de drapeau C5 est d'au moins environ 0,8 µM.
